# EUROPEAN PATENT SPECIFICATION

(11) **EP 4 426 247 B1**
(45) Date of publication and mention of the grant of the patent: **24.12.2025**
(21) Application number: 22803054.0
(22) Date of filing: 04.11.2022
(51) Int. Cl.: A61F 5/44

(54) **A VALVE FOR AN OSTOMY APPLIANCE**
VENTIL FÜR EINE OSTOMIEVORRICHTUNG
SOUPAPE POUR UN APPAREIL DE STOMIE

(30) Priority: 05.11.2021 GB 202115967; 05.11.2021 GB 202115966; 05.11.2021 GB 202115965; 22.02.2022 GB 202202404
(43) Date of publication of application: 11.09.2024
(73) Proprietor: Salts Healthcare Limited, West Midlands B7 4AA (GB)
(72) Inventor: HOWARD, Lee, Birmingham, West Midlands B7 4AA (GB); SMITH, Lee, Birmingham, West Midlands B7 4AA (GB); WILLIAMS, Kieran, Birmingham, West Midlands B7 4AA (GB)
(74) Representative: Forresters IP LLP
(86) International application number: PCT/GB2022/052796
(87) International publication number: WO 2023/079305

(56) References cited:
- DE-A1- 102014 104 737
- US-A1- 2012 286 187

## Description

### Introduction

Embodiments of the invention relate to a valve for an ostomy appliance or a urostomy appliance. Ostomy appliances are well known in the field. They are typically attached to a user via an adhesive member that extends around the user's stoma with adhesive and provide a collecting volume to collect waste (mostly liquid waste) exiting the stoma. A mechanism for draining the collecting volume is often provided - typically, these are in the form of a tap, so that a user can empty waste that is collected in the collecting volume without having to remove and dispose of the entire appliance.

DE102014104737 discloses an ostomy bag including a valve with a valve bushing which forms an outlet opening from the bag cavity and a valve plug with which the outlet opening can be closed.

According to a first aspect of the invention we provide a valve for an ostomy appliance according to the subject-matter of claim 1.

According to a second aspect of the invention we provide an ostomy appliance including:
a first and a second wall, which are connected about a periphery to provide an internal collecting volume;
a valve which is fluidly connected to the internal collecting volume for selectively permitting liquid to exit; the valve including the features according to the first aspect. according to the subject-matter of claim 11.

Further optional features relating to the aspects of the invention are outlined in the appended claims.

In order that the present disclosure may be more readily understood, preferable embodiments thereof will now be described, by way of example only, with reference to the accompanying drawings, in which:
FIGURE 1 is a view of an ostomy appliance;
FIGURE 2 is a perspective partial view of an ostomy appliance in accordance with an embodiment of the disclosure;
FIGURE 3 is another perspective partial view of an ostomy appliance;
FIGURE 4 is another perspective partial view of an ostomy appliance;
FIGURE 5 is another perspective partial view of an ostomy appliance;
FIGURE 6 is a perspective partial view of an ostomy appliance and a connected drainage tube;
FIGURE 7 is a perspective view of a valve for an ostomy appliance;
FIGURE 8 is a partial view of an ostomy appliance in accordance with an embodiment of the disclosure;
FIGURE 9 is a perspective view of a valve for an ostomy appliance;
FIGURE 10 is another perspective partial view of an ostomy appliance;
FIGURE 11 is a perspective partial view of an ostomy appliance and a connected drainage tube;
FIGURES 12A and 12B illustrate a valve for an ostomy appliance (figure 12B has a part cut away); and
FIGURES 13A and 13B illustrate a valve for an ostomy appliance.

Referring to the figures, an ostomy appliance 10 (appliance 10) is illustrated. The ostomy appliance 10 includes a first wall 12 which has an attachment member (not shown) for attaching the appliance 10 to the user and also defines a stoma receiving opening for fitting about the stoma of the user. A second wall 14 is connected about its periphery to a periphery of the first wall 12, so that between the first and second wall 12, 14 forms a collecting volume (to receive waste from the stoma).

The ostomy appliance 10 further includes an outlet portion 20. The outlet portion 20 is formed from a first and a second outlet wall 22, 24. The first and second outlet wall 22, 24 are connected along a periphery to form a tube / conduit. It should be appreciated that the outlet portion 20 could be formed from an extension / the same sheets as the first and second walls 12, 14.

The appliance 10 also includes a valve 100 which provides an outlet 102. The valve 100 is connected to the outlet portion 20. Thus, the outlet portion 20 and the valve 100 provide a flow path between the collecting volume and the outlet 102. In other words, the outlet portion 20 forms a conduit between the collecting volume and the valve 100. Waste flowing from the collecting volume will pass through the outlet portion 20 and through the valve 100 to exit the appliance 10 through the outlet 102.

The ostomy appliance 10 is specifically a urostomy or ileostomy appliance, which is configured to accommodate waste with a higher proportion of liquid than other ostomy appliances. Hence, the valve 100 must be able to prevent liquid from exiting the appliance 10 when it is in its closed position. Likewise, any other flow control measures put in place (see towards the end of the description) for the user to alter the flow out of the appliance 10 must also be suitable for waste containing a high percentage of liquid (i.e. waste with a relatively low viscosity which means the waste can flow in a similar manner to water or other liquid).

The valve 100 includes a body 110, an inlet connecting to the outlet 102 by a flow path, and a closure arrangement 120. The closure arrangement 120 is moveable between an open position and a closed position (and vice versa). In the closed position, the closure arrangement 120 blocks the outlet 102, such that liquid cannot flow through the outlet 102. In the open position, the outlet 102 is open, such that liquid is permitted to flow out of the outlet 102.

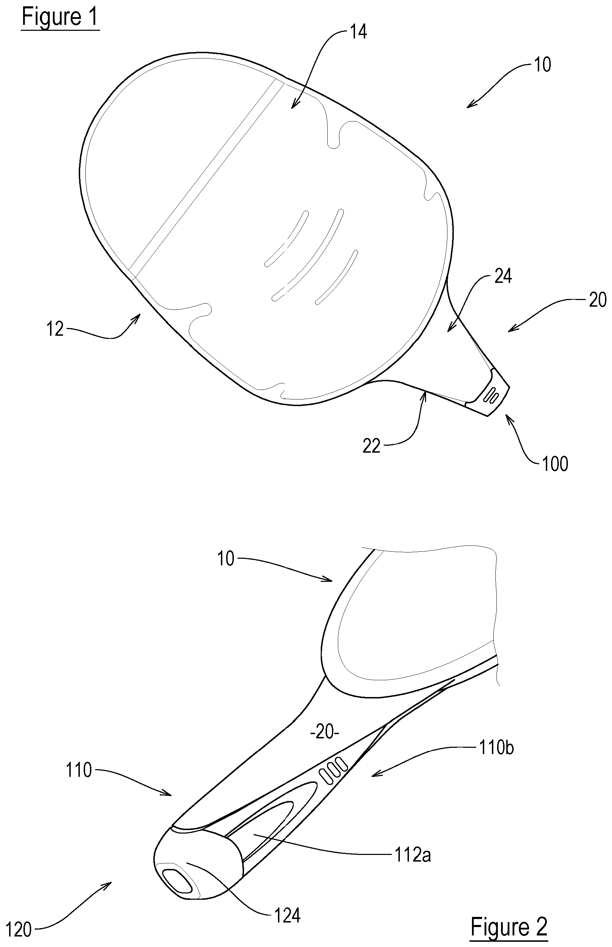

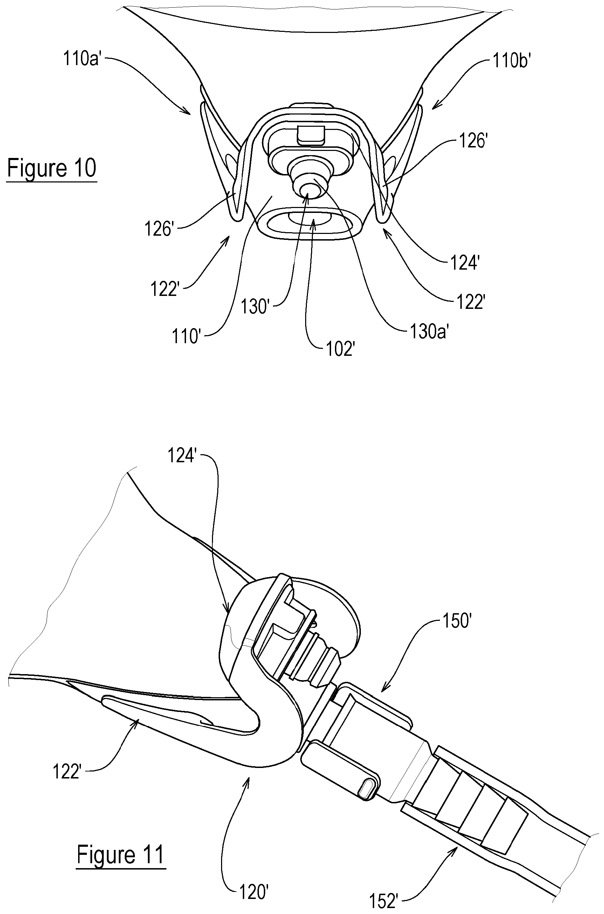

In some embodiments, one or more external surfaces of the valve 100 cooperate with one or more external surfaces of the outlet portion 20 to form a smooth transition between the outlet portion 20 and the valve 100 (see for example, figures 2 and 11). In other words, the valve 100 is incorporated into the outlet portion 20 to form a tail arrangement. Such incorporation is more comfortable for a user to wear. For example, it may reduce sharp corners and parts of the appliance 10 from digging in to a user's flesh, etc. In some embodiments, the outlet portion 20 provides the upper and lower external surfaces (of the tail arrangement) and the valve 100 provides both left and right side external surfaces. In some embodiments, the valve 100 is made of a soft and / or flexible material (e.g. to further increase the comfort of a user). More detail of how the valve is attached to an appliance is discussed below once the functionality of the valve has been discussed.

Features of the valve 100 will now be described in more detail. All embodiments discussed here may be combined with the ostomy appliance 10 structure described above.

As described above, the valve 100 includes a body 110, an inlet, an outlet 102 and a closure arrangement 120. The body 110 is configured to be connected to the ostomy appliance 10 (and may be attached to an outlet portion 20 as described above). The inlet and the outlet 102 are connected by a flow path (i.e. the flow path for waste out of the valve 100 and the appliance that the valve 100 is attached to). As mentioned above, the closure arrangement 120 is moveable between an open and a closed position in order to open and close the valve 100.

In some examples (such as those illustrated in figures 2 to 7), the closure arrangement 120 is detachably connectable to the body 110. In other words, the closure arrangement 120 is removable from the body 110 of the valve 100 and re-attachable as desired. Thus, the closure arrangement 120 may be attached to and supported by the body 110 irrespective of whether the valve 100 is open or closed.

In some embodiments, the closure arrangement 120 includes a connection member 122 that provides the detachable connection to the body 110. In the illustrated example, the closure arrangement 120 includes a closure body 124 and the connection member 122.

In some embodiments, the connection member 122 includes an elongate portion 126 that extends away from the closure body 124 (and that provides the connection to the body 110). Thus, the connection member 122 provides a projection that can be attached to the body 110 of the valve 100.

The closure body 124 supports a bung portion 130 that is configured to be positioned in the outlet 102 when the closure arrangement 120 is in its closed position (i.e. blocking waste from exiting the valve 100). Thus, the bung portion 130 is held in the outlet by a friction or push fit. The closure body 124 (and more specifically, the bung portion 130) is configured to be insertable into the outlet 102 of the valve 100 whether the connection member 122 is attached to the body 110 or not.

In some embodiments, the bung portion 130 includes a flanged part 130a that engages an internal surface inside the outlet 102. This may aid the connection between the closure and the body 110 of the valve and reduce the likelihood that the bung potion 130 will detach itself / come out of the outlet 102 accidently.

In some embodiments, the body 110 provides a first engagement formation 112 configured to receive a portion of the connection member 122. The connection member 122 also includes a corresponding engagement formation 128 that is configured to engage with the first engagement formation 112.

In the present example, the first engagement formation 112 is a receiving portion. In other words, the body 110 includes a recess or opening that "receives" a portion of the connection member 122 to hold and support the closure body relative to the body 110. The corresponding engagement formation 128 includes a projection that is configured to engage the recess or opening on the body 110. The recess or opening is positioned on a first side 110a of the body 110 and spaced "rearward" of the outlet 102 (i.e. towards the collecting volume of the appliance or upstream of the outlet 102).

In some embodiments, the first engagement formation 112 includes a channel 112a that leads to the recess or opening. The channel 112a provides an additional receiving portion that receives the connection member 122 (in this example, the elongate member 126 of the connection member 122), when the corresponding engagement formation 128 is received in the first engagement formation 112. Thus, when the connection member 122 is connected to the body 110, an external surface of the body 110 is in line with an external surface of the connection member 122 to provide a smooth or continuous external surface. This is advantageous because it reduces the possibility that the connection member 122 will catch on external items and become inadvertently detached from the body 110 (this is especially true when a user is using the appliance 10 and not aware that the detachment has happened because they are not using the valve to empty the appliance 10).

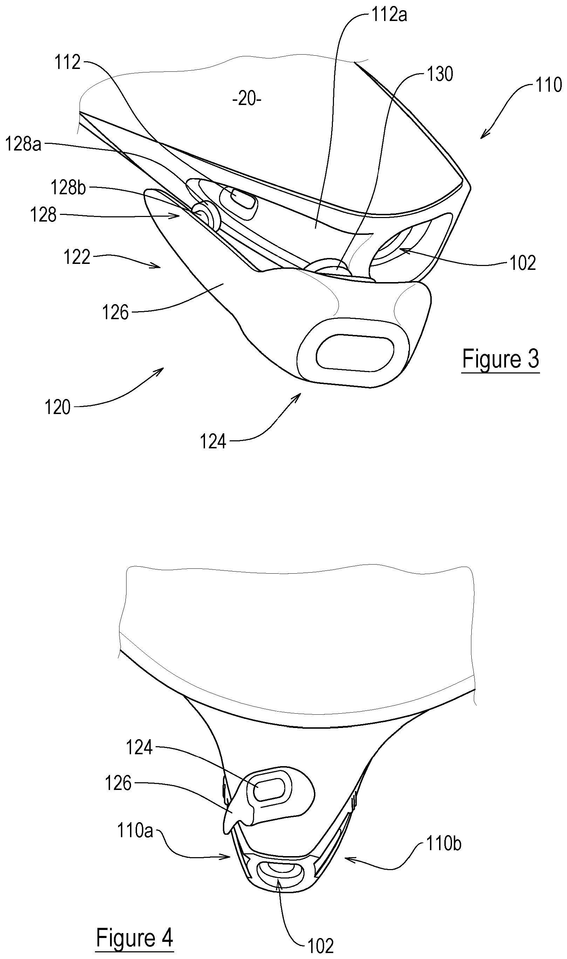

In some embodiments, the connection between the closure arrangement 120 and the body 110 permits rotation of the closure arrangement 120 while it remains attached. In other words, the connection member 122 can be rotated while the engagement formation on the body 110 and the corresponding engagement formation are engaged / connected. In the illustrate example (see particularly figure 3 for a clear view), the corresponding engagement formation 128 of the connection member 122 includes a projection with a domed portion 128a and a narrowed section 128b. The first engagement formation 112 includes a recess wherein the opening at the surface is narrowed with respect to the width of the recess behind. In other words, the opening forms a pinch point. At least one of the domed portion 128a and the material of the first engagement formation 112 is formed of a resiliently deformable material. Thus, when the domed portion 128a is inserted through the opening and into the recess, the opening or the domed portion deforms to permit the domed portion 128a through the opening, and then returns to its initial form / shape. Having returned to their initial forms, the material forming the opening resists movement of the domed portion 128a outwards (i.e. away from the body 110) but allows the narrowed portion to rotate while maintaining the domed portion 128a within the recess. Therefore, the first engagement formation 112 resists removal of the corresponding formation 128 but allows the closure body to rotate with respect to the body 110.

In some embodiments, the closure arrangement 120 is connectable to the body 110 in two different locations. In other words, the connection member 122 may be attached to the body 110 in a choice of at least two locations or positions. These connection options are in addition to the required positioning of the bung portion 130 with respect to the outlet 102 to close the valve 100 (e.g. the bung portion 130 / closure body 124 is always receivable in the outlet 102 in order to move the closure arrangement 120 to its closed position).

In such an example, the body 110 includes a second engagement formation. Thus, the corresponding engagement formation 128 can engage either the first or the second engagement formation. In this example, the second engagement formation has similar or the same form as the first engagement formation 112, so that the corresponding engagement formation 128 of the connection member 122 can engage either of the engagement formations as desired by a user.

In some embodiments, the first receiving formation 112 is located on a first side 110a of the body 110 and the second receiving formation is located on a second side 110b. In the present example, the first and second sides are on opposing sides of the body 110. Thus, the connection member 122 may engage either the first side 110a or the second side 110b of the body 110.

In embodiments where the first and second engagement formations both include the channel 112a that extends towards the recess or opening, the other one of the engagement formations 112 that is not being used by the connection member 122 also has a function. The corresponding engagement formation 128 and the connection member 122 are received in the channel and recess, respectively, to form a substantially continuous external surface. On the opposing side 110a or 110b the channel of the "other" of the first and second engagement formations provides a usable notch for the user to open the valve 100 (i.e. it provides a space for the user to grasp the closure body 124 and to pull it outwards from the outlet 102 and move the closure arrangement 120 to its open position).

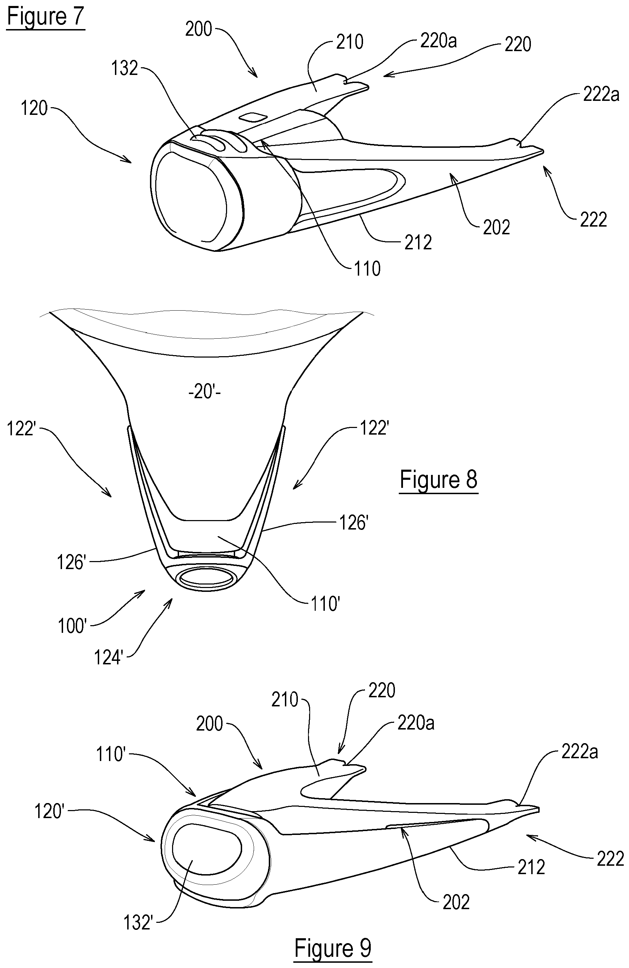

In some embodiments, the closure body 124 includes a gripping portion 132 to aid a user moving the closure arrangement 120 to its open position and / or closed position. In the illustrated example (e.g. see figure 7), the gripping portion 132 includes a raised surface which provides a surface that is easier to grip than the smooth surface of the remainder of the closure body 124.

In some embodiments, at least the connection member 122 (and more specifically the elongate portion 126) is formed from an elastic / resiliently deformable material, which allows the connection between the closure arrangement and the body to be flexible and resist permanent deformation.

In some embodiments, the valve 100 (i.e. the body 110 and the closure arrangement 120) is formed from a single material (preferably a soft / pliable or flexible material - e.g. TPE - to provide a comfortable wearing experience for the user).

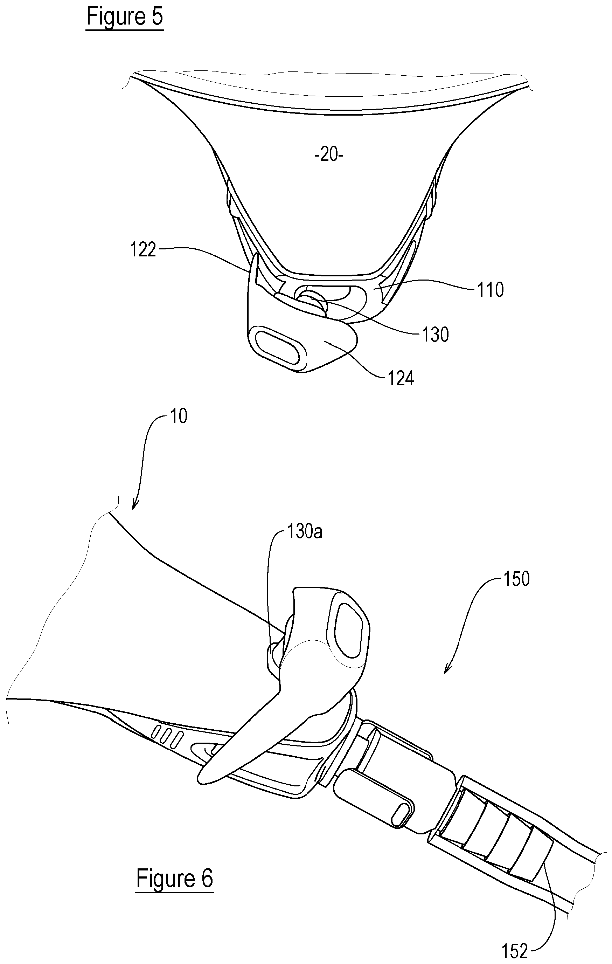

In use, the valve 100 is opened by moving the closure arrangement 120 to its open position. Essentially, this must involve unblocking the outlet 102. Thus, the closure body 124 is moved out of contact with the outlet 102. In addition, the closure arrangement 120 may be detached from the body 110 of the valve 100 (this is shown in detail in figure 3). In some embodiments, the ability to rotate the closure arrangement 120 may also be provided, as discussed above. The ability to leave the closure arrangement 120 attached to the body 110 of the valve 100 while opening the outlet 102 is shown in more detail in figures 4 and 5. Figure 5 shows when the closure body 124 has initially been removed from the outlet 102 - the connection member 122 can be seen flexing while maintaining the connection with the body 110 so that the closure body 124 can be moved away from the outlet 102. Figure 4 shows the closure arrangement 120 rotated up and away from the outlet 102.

If the user is attaching an accessory 150 to their appliance 10 (see, for example, figure 6), the closure arrangement 120 may be moved out of the way and remains connected to the body 110 of the valve 100. Alternatively, the closure arrangement 120 may be removed completely and stored elsewhere while the accessory 150 is in use. Figure 6 illustrates the attachment of a so called "night drainage adaptor" which allows the user to connect a separate tube 152 to their appliance 10 and drain waste into a second collecting vessel to extend the period of time between emptying the appliance 10 (this is particularly useful at night when someone will want to sleep for an extended period of time). Being able to remove the closure arrangement 120 may improve comfort, as the addition material of the valve 100 can be placed elsewhere (not close to the body).

A second embodiment of a valve is illustrated in figures 8 to 11. Unless explicitly stated, optional features that relate to the valve illustrated in figures 2 to 7 are equally useable on the valve in figures 8 to 11. Where features of the valve or appliance are the same or similar to those already discussed above, they are provided with the same reference number with a prime symbol (i.e. 10 will become 10').

The valve 100' includes the body 110', the inlet, the outlet 102' and the closure arrangement 120' as already discussed. Again, the closure arrangement 120' is moveable between a closed position and an open position. Where, in the closed position, the closure arrangement 120' blocks the outlet 102', such that liquid cannot flow through the outlet 102'. And, in the open position, the outlet 102' is open, such that liquid is permitted to flow out of the outlet 102'.

In some embodiments, the closure arrangement 120' is connectable / connected to the body 110' in two locations (see particularly figures 8 and 10). In other words, the closure arrangement 120' is connected either permanently or removably in two places on the body 110'. In the illustrated examples, the connections members 122' are permanently attached to the body 110'.

In some embodiments, the closure arrangement 120' includes first and second connection members 122'. Each of the first and second connection members 122' connects to the body 110'. In the illustrated example, the closure arrangement 120' includes a closure body 124' and first and second connection members 122'.

In some embodiments, each connection member 122' includes an elongate portion 126' that extends away from the closure body 124'. In some embodiments (for example, figures 8 onwards), the closure body 124' is generally central and between the two connection members 122'. In other words, the first connection member 122' connects to a first side of the closure body 124' and the second connection member 122' connects to a second side of the closure body 124'. The connection members 122' are on opposing sides of the closure body 124'.

In some embodiments, the first connection member 122' connects to a first side 110a' of the body 110' and the second connection member 122' connects to a second side 110b' of the body 110'. Thus, the connections members 122' are on opposing sides of the body 110' of the valve 100'. In the present example, the connection members 122' forms the external "sides" of the valve 100' - in that the first connection member 122' extends from the first side of the closure body 124' to the first side 110a' of the body 110' and the second connection member 122' extends from the second side of the closure body 124' to the second side 110b' of the body 110'.

Further, in this example, again the closure body 124' supports a bung portion 130' that is configured to be positioned in the outlet 102' when the closure arrangement 120' is in its closed position (i.e. blocking waste from exiting the valve 100'). Thus, the bung portion 130' is held in the outlet 102' by a friction or push fit. In this embodiment, the first and second connection members 122' provide additional biasing force to urge the closure body 124' (and the bung 130') towards the closed position when the closure body is aligned or substantially aligned with the outlet 102'.

Like the single connection member valve version described above, the bung portion 130' includes a flanged part 130a' that engages an internal surface inside the outlet 102'. This may further reinforce the connection between the closure and the body 110' of the valve and reduce the likelihood that the bung potion 130' will detach itself / come out of the outlet 102' accidently.

In some embodiments, the closure body 124' includes a grasping or user aid 132' that is formed to assist the user when grasping and / or moving the closure body 124'. The aid 132' may include a recess / detent for a finger, and / or raised ridges to provide additional grip for the user.

The way in which the valve 100' functions will now be discussed as this is different to the valve 100 described above due to the two connection points between the closure arrangement 120' and the body 110'.

As already mentioned, when the valve 100' is closed (i.e. the closure arrangement 120' is in its closed position), which is illustrated on figures 8 and 9, the closure body 124' is generally aligned with the outlet 102' (which allows the bung portion 130' to be inserted into the outlet 102' to close the valve 100'). In some embodiments, the first and second connection members 122' are formed of a resiliently deformable or biased material. This means that in order to remove the closure body 124' bung portion 130' from the closed position (i.e. move them outwards and away from the body 110'), the connection members 122' are stretched from their original form. Thus, the connection members 122' act to resist removal of the closure body 124' from the closed position. In some embodiments, the connection members 122' apply an additional force on the closure body 124' when in the closed position - thus, the connection members 122' "pull" the closure body 124' into closer contact with the body 110' (i.e. the connections members 122' are deflected from their natural shape / size / configuration and apply a biasing force towards the body 110').

When the closure arrangement 120' is in its open position, it is held or positioned adjacent the body 110'. In some embodiments (see, for example, figures 10 and 11), the closure arrangement 120' is held in a position that contacts the body 110' when it is in its open position.

To move the closure body 124' from alignment with the outlet 102' (i.e. closed), the closure body 124' can be pulled away from the body 110' and / or transverse force is used to roll the closure body 124' towards a front or back of the body 110'. This movement is against the pull of the connection members 122' on each side of the closure body 124'. A result of this movement means that the closure body 124' can be urged away from the outlet 102'.

In some embodiments, the closure body 124' is moved to an offset position, out of alignment with the outlet 102'. The first and second connection members 122' are deflected and hold the closure body 124' against / in contact with an external surface of the body 110'. As above, because the connection members 122' are made of a resiliently deformable material, they are deflected from their natural configuration and thus apply a biasing force to the closure body 124' towards the side of the body 110'.

When a user wishes to close the valve 100', the closure body 124' must be actively moved from its open position towards the outlet 102' (i.e. a force must be applied to the closure arrangement 120' when it is in the open position to return it to the closed position). This is advantageous because the closure arrangement 120' is held stably in a well-defined / predetermined position away from the outlet in the open position (and against the body 110' of the valve). Previous designs have often relied on a closure being allowed to fly in free space when in the open position, whereas this closure body is held in a position where it will not interfere with further use of the valve 100'.

If the user is attaching an accessory 150' to their appliance 10' (see, for example, figure 11), the closure arrangement 120' may be moved out of the way and is held against the side of the body 110'. Figure 11 illustrates the attachment of a night drainage adaptor which allows the user to connect a separate tube 152' to their appliance 10' and drain waste into a second collecting vessel to extend the period of time between emptying the appliance 10.

Having a predetermined position in which the closure body 124' is held when in the open position improves comfort for the user because it will not hang away from their appliance 10' in a potentially uncomfortable position.

Additional features of the valve will now be described that have an impact on how the valve 100, 100' is attached to an ostomy appliance 10, 10'. Figures 7 and 9 illustrate valves that have the features described above already and are illustrated alone and not attached to any appliance 10, 10'. Where features of the valve are discussed, only the reference number without a prime has been used for clarity. However, it should be appreciated that the valve references with a prime symbol are equally applicable.

The body 110 includes a first attachment portion 200 and a second attachment portion 202. Each attachment portion 200, 202 extends away from the inlet. In other words, the valve 100 includes a pair of attachment portions 200, 202 which are elongate and project away from the body 110 of the valve 100. In a top down or bottom up view of the valve 100, the attachment portions 200, 202 extend away from the body 110, so that the overall valve shape is generally U or V shaped (i.e. each attachment portion 200, 202 forms a limb).

The first and second attachment portions 200, 202 provide one or more attachment surfaces for connection to the ostomy appliance 10. In the illustrated examples, the attachment portions 200, 202 provide first and second attachment surfaces 210, 212. The first attachment surface 210 lies substantially in a first plane and the second attachment surface 212 lies substantially in a second plane. The first plane and the second plane are inclined with respect to each other.

In some embodiments, the first attachment surface 210 is formed from an external surface of the body 110. The second attachment surface 212 is formed from another external surface of the body 110. In the illustrated example, the external surfaces forming the first and second attachment surfaces are on opposing sides of the valve 100. In other words, the first attachment surface 210 forms an upper surface of the valve 100 and the second attachment surface 212 forms a lower surface of the valve 100.

In this example, the inlet and outlet 102 and the flow path extending between them extends through a generally central portion of the body 110. Thus, the generally central portion of the body extends between the first and second attachment surfaces 210, 212. In other words, (upper and lower) external surfaces of the generally central portion of the body and the first and second attachment portions 200, 202 form the first and second attachment surfaces 210, 212.

In some embodiments, the first and or the second attachment portions 200, 202 taper (i.e. get progressively narrower) as they extend away from the central portion of the body of the valve 100. In other words, the first and second attachment portions 200, 202 each form a general wedge shape when viewed side on. Thus, the first and second planes in which the first and second attachment surfaces 210, 212 generally extend intersect to form a point where the valve ends. In other words, the first and second attachment surfaces 210, 212 meet at an end of first attachment portion 200 and form a first tip 220 (i.e. at one of the ends of the U / V shape). Likewise, at the second attachment portion 202, the first and second attachment surfaces 210, 212 meet to form a second tip 222 (on the other end of the U / V shape).

In some embodiments, one or both of the first and second tips 220, 222 includes a notched portion 220a, 222a. This is advantageous because it aids the manufacturing process and makes it easier to achieve a seal between outlet portion 20, 20' material and the attachment surfaces 210, 212 The valve 100 is attached to the outlet portion 20. In the present example, the first attachment surface 210 is connected to the first outlet wall 22 and the second attachment surface 212 is connected to the second outlet wall 24. Preferably, the outlet portion 20 is heat-welded to the first and second attachment surfaces 210, 212 as this provides a secure and reliable seal between the components.

Thus, the tail arrangement includes the outlet portion 20 and the valve 100. Internally, the passage for waste (prior to entering the flow path of the valve 100) is formed from the first and second outlet walls 22, 24 and the first and second attachment portions 200, 202 of the valve 100. At a top part of the outlet portion 20, the first and second outlet walls 22, 24 are attached together. Then, as the valve begins (further away from the collecting volume), the first and second tips 220, 222 are included into the "wall" (i.e. the first and second outlet walls are no longer attached to each other and are attached to the first and second attachment surfaces 210, 212 instead). As more of the valve 100 is incorporated (i.e. closer to the inlet and outlet 102), the attachment portions 200, 202 of the body 110 become gradually wider. Thus, the sides of the tail arrangement get gradually wider on the approach to the inlet / outlet 102. This configuration provides the smooth tapering of the surface of the appliance 10 as previously described.

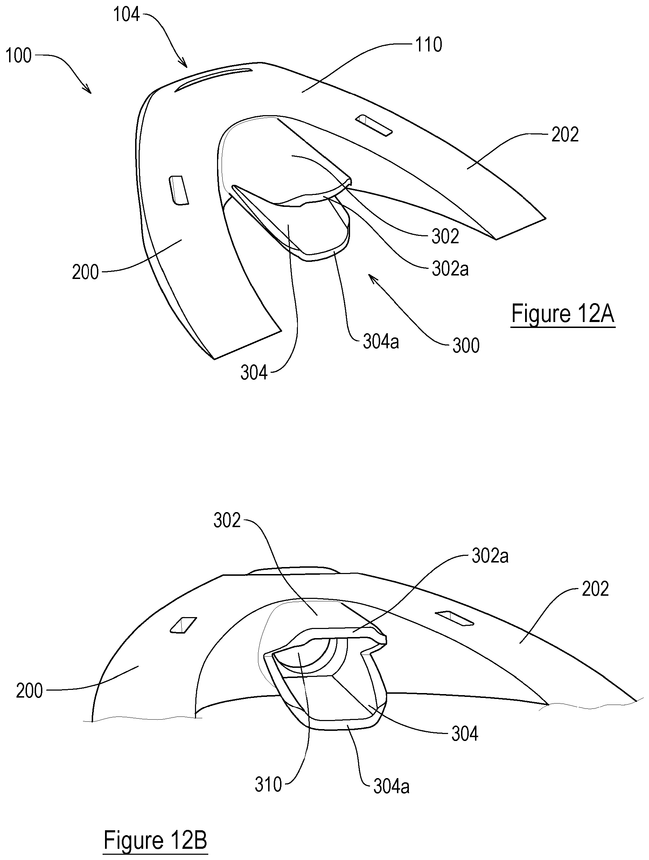

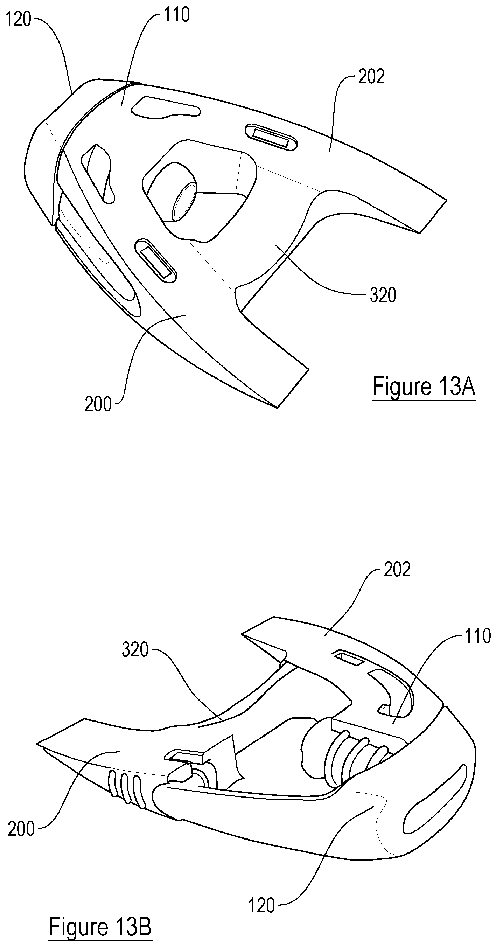

According to the invention, the valve 100 includes a flow control formation 300 (see figures 12 and 13).

The flow control formation 300 is configured to default to an open condition and has a closed condition in which the flow of liquid from the outlet 104 is prevented or at least inhibited. In others words, the flow control formation 300 remains open (and allows waste through to the outlet) unless it is moved to the closed condition - in some examples, the flow control formation 300 is moved to its closed condition by a user manipulating it.

It should be appreciated that the flow control formation 300 could be provided in any of the valves 100, 100' discussed above. According to the invention, the flow control formation 300 is located internally to the appliance 10 (i.e. within the tail arrangement formed by the outlet portion 20 and the valve 100). The internal portions of the flow control formation 300 allow more effective manual flow control by a user.

The figures illustrate two different configurations of the flow control formation 300. The example illustrated in figure 12A and 12B is described first. The flow control formation 300 has a deflectable part 302 and a further, second deflectable part 304. The first and second deflectable parts 302, 304 are moveable towards each other in order to close the flow path towards the outlet 104. It should be appreciated that there may be a single deflectable part that moves to close the flow path against another surface of the valve 100 or against another part of the appliance 10.

The first and second deflectable parts 302, 304 are located internally to the appliance 10. In this example, the first and second deflectable parts 302, 304 are resiliently biased to an initial shape and / or position (which is shown in figure 12A). This initial shapes and / or position corresponds to the open condition of the flow control formation 300.

In this illustrated example, the first and / or the second deflectable parts 302, 304 project from the body 110 of the valve 100. In other words, the deflectable parts 302, 304 extend "upstream" of the body 110 and outlet 104.

The first and second deflectable parts 302, 304 are located around an opening 310 in the body 110 that leads to the outlet 104. The first deflectable part 302 extends around half of the opening and extends away / upstream from the body 110 (i.e. it forms an upper beak). The second deflectable part 304 extends around the second half of the opening and also extends away / upstream forms the body 110 (and forms a lower beak).

In their "normal" or default position, the ends 302a, 304a of the first and second deflectable parts 302, 304 that are furthest from the opening in the body 110 are spaced from each other and provide an open passage for liquid / waste to flow through (whether the waste flows out of the valve 100 is down to the position of the closure arrangement 120 (not shown in figures 12A and 12B)).

In use (i.e. when a user wishes to influence the flow of waste through the valve without using the closure arrangement 120), the deflectable parts 302, 304 are moved towards each other (i.e. opposing pressure is placed on each "side"). The ends 302a, 304a are moved into contact with each other. The upper and the lower beak contact each other along the sides in addition to the end and the flow path is closed (i.e. to at least some extent the pressure on either side of the beak allows the sides / end to for a temporary seal and prevent or at least inhibit liquid flowing to the opening 310).

The flow control formation 300 is maintained in this closed condition for as long as pressure is applied to hold the deflectable parts 302, 304 together. Thus, once a user is happy to allow liquid to flow again, the pressure is released and the flow path is opened again.

The invention illustrated in figures 13A and 13B will now be described. The flow control formation 300 includes a contact surface 320 upstream of the outlet 104 (i.e. the contact surface 320 is located between the internal collecting volume of the appliance 10 and the outlet 104 of the valve 100). Further, in the present example, the contact surface 320 is internal to the appliance 10 (i.e. between the layers of the outlet portion 20). In the present example, the contact surface 320 extends from one attachment portion 200 of the body 110 to the second attachment portion 202. Thus, the contact surface 320 extends across the space formed between the limbs of the body 110.

In one example, the contact surface 320 includes a convex portion (preferably the convex portion is generally central with respect to the contact surface 320 and generally aligned with an opening in the body 110 that connects to the outlet 104). In the illustrated example, the contact surface 320 is convex on one side but it should be appreciated that both sides could include a convex portion.

The valve 100 is positioned / attached between the first and second outlet walls 22, 24 (although it should be appreciated that is the appliance 10 is made with the outlet portion 20 integrated into the first and second walls 12, 14 then the valve 100 will be positioned / attached between the first and second walls 12, 14 instead). All that matters for the contact surface 320 is that the flexible membrane (i.e. the layers provided by the walls 12, 14, 22, 24) of the appliance 10 extends either side of the contact surface 320 to provide a flow path between the internal collecting volume and the outlet 104.

If there is no interaction from a user or other pressure on the contact surface 320, the flow path between the collecting volume and the outlet 104 of the valve 100 remains open (i.e. whether liquid is permitted to flow out of the valve is determined by the closure arrangement 120 / whether the outlet 104 is open or closed).

In use (i.e. when a user wishes to influence the flow of waste through the valve 100 without using the closure arrangement 120), compressive force is applied to one or both sides of the contact surface 320 to close the flow path. In other words, the film of the walls of the appliance 10 are pinched against the contact surface 320 and the flow path to the outlet 104 is blocked. The compressive force / pressure may be applied by the user, for example, a finger and a thumb can be used to pick the layers of film against the contact surface 320 to stem flow of liquid. Thus, the convex portion of the contact surface 320 may be advantageous as it permits the curve of a digit (i.e. the thumb / finger of the user of the appliance 10 or a nurse providing assistance) to pinch the layers more efficiently and / or more comfortably.

The flow path remains closed and preventing (or at least inhibiting) the flow of liquid for as long as the layers are pressed against the contact surface 320. Once the closed condition is no longer required, the pressure is released and the layers (the first and second walls 12, 14 / first and second outlet walls 22, 24) are pushed away from the contact surface 320 by the liquid flowing to the outlet 104 (e.g. under gravity).

In embodiments, the flow control formation 300 is advantageous because it allows manual alteration of the flow of liquid from the valve 100 irrespective of the closure arrangement 120. Thus, a user wishing to empty their appliance 10 can keep the valve 100, 100' "closed" even after the closure arrangement 120 has been moved to its open position using the control flow formation 300. This means that the user can stop the immediate flow of liquid from the outlet 104 after opening the closure arrangement 120 - thus, avoiding undesired waste flowing over fingers, clothes or other surfaces before they have oriented the valve 100 to direct the flow into a toilet or other receptacle. This provides a cleaner and more dignified experience for someone using the appliance 10.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Although certain example embodiments of the invention have been described, the scope of the appended claims is not intended to be limited solely to these embodiments. The claims are to be construed literally, purposively, and/or to encompass equivalents.

## Claims

1. A valve (100) for an ostomy appliance (10) including:
a body (110) for connection to the ostomy appliance (10),
an inlet and an outlet (104), connected by a flow path,
a closure arrangement (120) which is moveable between a closed position, in which the closure arrangement blocks the outlet (104), such that liquid cannot flow through the outlet (104), and an open position in which the outlet (104) is open, such that liquid is permitted to flow out of the outlet (104), and **characterised in that** the valve (100) includes
a flow control formation (300), which includes a contact surface (320) upstream of the outlet (104) and is configured to be located internally to an attached ostomy appliance (10), and is configured to default to an open condition and has a closed condition in which the flow of liquid from the outlet (104) is prevented or at least inhibited.

2. A valve (100) according to claim 1 wherein the flow control formation (300) is moveable to its closed condition via manual manipulation by a user.

3. A valve (100) according to any one of the preceding claims wherein the flow control formation (300) has a deflectable part, which is moveable to close the flow path.

4. A valve (100) according to claim 3 wherein the flow control formation (300) includes a further, second deflectable part (304), so that the first and second deflectable parts (302, 304) are moveable towards each other to close the flow path.

5. A valve (100) according to claims 3 or 4 wherein the first and / or the second deflectable (302, 304) parts are resiliently biased to an initial shape and / or position, which corresponds to the open condition.

6. A valve (100) according to any one of claims 3 to 5 wherein the first and / or the second deflectable parts (302, 304) project from the body (110) of the valve (100).

7. A valve (100) according to any one of the preceding claims wherein the contact surface contacts a portion of the ostomy appliance when the flow path is closed.

8. A valve (100) according to any one of the preceding claims wherein the contact surface includes a convex portion.

9. A valve (100) according to any one of the preceding claims wherein the body (110) includes a first and a second attachment portion, each of which extends away from the inlet, and the contact surface extends between the first and second attachment portions.

10. A valve (100) according to any of the preceding claims wherein the body (110) forms a U or V shape.

11. An ostomy appliance (10) including:
a first and a second wall, which are connected about a periphery to provide an internal collecting volume;
a valve (100) which is fluidly connected to the internal collecting volume for selectively permitting liquid to exit; the valve (100) according to any of claims 1 to 10.

12. An ostomy appliance (10) according to claim 11 wherein the body (110) of the valve (100) is connected between the first and second walls (12).

13. An ostomy appliance according to claim 12 wherein the contact surface contacts the first wall and / or the second wall (12) to close the flow path or the ostomy appliance (10) further includes an outlet (104) portion provided between the internal collecting volume and the valve (100), said outlet (104) portion formed of first and second outlet walls (22, 24) and where one or both of the first and second outlet walls (22, 24) contact the contact surface to close the flow path.

14. An ostomy appliance according to claim 13 wherein compressive force is applied to one or both sides of the contact surface to close the flow path.

15. An ostomy appliance according to any one of claims 11 to 14wherein the body (110) includes first (210) and second (212) attachment surfaces and a generally central portion through which the flow path extends, and the first wall (12) attaches to the first attachment surface (210) and the second wall (12) attaches to the second attachment surface (212).

## Patentansprüche

1. Ventil (100) für eine Stomavorrichtung (10), umfassend:
Einen Körper (110) zur Verbindung mit der Stomavorrichtung (10),
einen Einlass und einen Auslass (104), die durch einen Strömungsweg verbunden sind,
eine Verschlussanordnung (120), die zwischen einer geschlossenen Position, in der die Verschlussanordnung den Auslass (104) blockiert, sodass keine Flüssigkeit durch den Auslass (104) fließen kann, und einer offenen Position, in der der Auslass (104) geöffnet ist, sodass Flüssigkeit aus dem Auslass (104) fließen kann, beweglich ist, und **dadurch gekennzeichnet, dass** das Ventil (100) umfasst:
eine Durchflussregelformation (300), die eine Kontaktfläche (320) stromaufwärts des Auslasses (104) umfasst und so konfiguriert ist, dass sie sich innerhalb einer angeschlossenen Stomavorrichtung (10) befindet, und so konfiguriert ist, dass sie standardmäßig einen offenen Zustand einnimmt und einen geschlossenen Zustand aufweist, in dem der Flüssigkeitsfluss aus dem Auslass (104) verhindert oder zumindest gehemmt wird.

2. Ventil (100) nach Anspruch 1, wobei die Durchflussregelformation (300) durch manuelle Betätigung durch einen Benutzer in ihren geschlossenen Zustand bewegt werden kann.

3. Ventil (100) nach einem der vorhergehenden Ansprüche, wobei die Durchflussregelformation (300) einen ablenkbaren Teil aufweist, der beweglich ist, um den Strömungsweg zu schließen.

4. Ventil (100) nach Anspruch 3, wobei die Durchflussregelformation (300) einen weiteren, zweiten auslenkbaren Teil (304) umfasst, sodass die ersten und zweiten auslenkbaren Teile (302, 304) aufeinander zu bewegt werden können, um den Strömungsweg zu schließen.

5. Ventil (100) nach Anspruch 3 oder 4, wobei der erste und/oder der zweite auslenkbare Teil (302, 304) durch Elastizität in eine Ausgangsform und/oder -position vorgespannt sind, die dem offenen Zustand entspricht.

6. Ventil (100) nach einem der Ansprüche 3 bis 5, wobei der erste und/oder der zweite auslenkbare Teil (302, 304) aus dem Körper (110) des Ventils (100) hervorragen.

7. Ventil (100) nach einem der vorhergehenden Ansprüche, wobei die Kontaktfläche einen Abschnitt der Stomavorrichtung berührt, wenn der Strömungsweg geschlossen ist.

8. Ventil (100) nach einem der vorhergehenden Ansprüche, wobei die Kontaktfläche einen konvexen Abschnitt umfasst.

9. Ventil (100) nach einem der vorhergehenden Ansprüche, wobei der Körper (110) einen ersten und einen zweiten Befestigungsabschnitt umfasst, die sich jeweils vom Einlass weg erstrecken, und die Kontaktfläche sich zwischen dem ersten und dem zweiten Befestigungsabschnitt erstreckt.

10. Ventil (100) nach einem der vorhergehenden Ansprüche, wobei der Körper (110) eine **U-** oder V-Form bildet.

11. Stomavorrichtung (10), umfassend:
Eine erste und eine zweite Wand, die um einen Umfang herum verbunden sind, um ein inneres Sammelvolumen bereitzustellen;
ein Ventil (100), das mit dem inneren Sammelvolumen in Fluidverbindung steht, um selektiv den Austritt von Flüssigkeit zu ermöglichen; das Ventil (100) nach irgendeinem der Ansprüche 1 bis 10.

12. Stomavorrichtung (10) nach Anspruch 11, wobei der Körper (110) des Ventils (100) zwischen der ersten und der zweiten Wand (12) angeschlossen ist.

13. Stomavorrichtung nach Anspruch 12, wobei die Kontaktfläche die erste Wand und/oder die zweite Wand (12) berührt, um den Strömungsweg zu schließen, oder die Stomavorrichtung (10) ferner einen Auslassabschnitt (104) umfasst, der zwischen dem inneren Sammelvolumen und dem Ventil (100) vorgesehen ist, wobei der Auslassabschnitt (104) aus ersten und zweiten Auslasswänden (22, 24) besteht und wobei eine oder beide der ersten und zweiten Auslasswände (22, 24) die Kontaktfläche berühren, um den Strömungsweg zu schließen.

14. Stomavorrichtung nach Anspruch 13, wobei auf eine oder beide Seiten der Kontaktfläche eine Druckkraft ausgeübt wird, um den Strömungsweg zu schließen.

15. Stomavorrichtung nach einem der Ansprüche 11 bis 14, wobei der Körper (110) eine erste (210) und eine zweite (212) Befestigungsfläche sowie einen im Allgemeinen zentralen Abschnitt aufweist, durch den der Strömungsweg verläuft, und die erste Wand (12) an der ersten Befestigungsfläche (210) und die zweite Wand (12) an der zweiten Befestigungsfläche (212) befestigt ist.

## Revendications

1. Soupape (100) pour appareil de stomie (10), comprenant :
un corps (110) destiné à être raccordé à l'appareil de stomie (10),
une entrée et une sortie (104), raccordées à un trajet d'écoulement,
un dispositif de fermeture (120) que l'on peut déplacer entre une positon fermée dans laquelle le dispositif de fermeture bloque la sortie (104), de sorte que le liquide ne puisse pas s'écouler à travers la sortie (104), et une position ouverte dans laquelle la sortie (104) est ouverte, de sorte que le liquide puisse s'écouler hors de la sortie (104), et **caractérisé en ce que** la soupape (100) comporte :
une formation de régulation d'écoulement (300), qui comporte une surface de contact (320) en amont de la sortie (104) et est configurée pour être située à l'intérieur d'un appareil de stomie en place (10) et configurée pour passer par défaut sur une condition fermée et qui présente une condition fermée dans laquelle l'écoulement de liquide, à partir de de la sortie (104), est évité ou au moins inhibé.

2. Soupape (100) selon la revendication 1, dans lequel un utilisateur peut manuellement faire passer la formation de régulation d'écoulement (300) sur sa condition fermée.

3. Soupape (100) selon l'une quelconque des revendications précédentes, dans lequel la formation de régulation d'écoulement (300) comporte une partie orientable pouvant être déplacée pour fermer le trajet d'écoulement.

4. Soupape (100) selon la revendication 3, dans lequel la formation de régulation d'écoulement (300) comprend une autre, une seconde, partie orientable (304), de sorte que les première et seconde parties orientables (302, 304) peuvent être déplacées l'une vers l'autre pour fermer le trajet d'écoulement.

5. Soupape (100) selon soit la revendication 3, soit la revendication 4, dans lequel la première et/ou la seconde parties orientables (302, 304) sont élastiquement ramenées vers une forme et/ou une position initiale qui correspond à la condition ouverte.

6. Soupape (100) selon l'une quelconque des revendications 3 à 5, dans lequel la première et/ou la seconde parties orientables (302, 304) font saillie hors du corps (110) de la soupape (100).

7. Soupape (100) selon l'une quelconque des revendications précédentes, dans lequel la surface de contact entre en contact avec une partie de l'appareil de stomie lorsque le trajet d'écoulement est fermé.

8. Soupape (100) selon l'une quelconque des revendications précédentes, dans lequel la surface de contact est dotée d'une partie convexe.

9. Soupape (100) selon l'une quelconque des revendications précédentes, dans lequel le corps (110) comprend une première et une seconde parties de fixation, chacune d'entre elles partant de l'entrée, et la surface de contact se prolonge entre la première et la seconde parties de fixation.

10. Soupape (100) selon l'une quelconque des revendications précédentes, dans lequel le corps (110) forme une forme en U ou en V.

11. Appareil de stomie (10), comprenant :
une première et une seconde parois, qui sont raccordées autour d'une périphérie pour fournir un volume de collecte interne ;
une soupape (100) qui est fluidiquement raccordée au volume de collecte interne pour sélectivement permettre au liquide de s'échapper ; la soupape (100) selon l'une quelconque des revendications 1 à 10.

12. Appareil de stomie (10) selon la revendication 11, dans lequel le corps (110) de la soupape (100) est raccordé entre la première et la seconde parois (12).

13. Appareil de stomie selon la revendication 12, dans lequel la surface de contact entre en contact avec la première paroi et/ou la seconde parois pour fermer le trajet d'écoulement, ou l'appareil de stomie (10) comprend en outre une partie de la sortie (104) prévue entre le volume de collecte interne et la soupape (100), ladite partie de la sortie (104) étant formée de la première et de la seconde parois (22, 24), et où une ou les deux des première et seconde parois de la sortie (22, 24) entrent en contact avec la surface de contact pour fermer le trajet d'écoulement.

14. Appareil de stomie selon la revendication 13, dans lequel une force de compression est appliquée sur un ou sur les deux côtés de la surface de contact pour fermer le trajet d'écoulement.

15. Appareil de stomie selon l'une quelconque des revendications 11 à 14, dans lequel le corps (110) comprend une première (210) et une seconde (212) surfaces de fixation et une partie généralement médiane, à travers laquelle se prolonge le trajet d'écoulement, et la première paroi (12) se fixe à la première surface de fixation (210) et la seconde paroi (12) se fixe à la seconde surface de fixation (212).
